# EUROPÄISCHE PATENTSCHRIFT

(11) **EP 1 884 254 B1**
(45) Veröffentlichungstag und Bekanntmachung des Hinweises auf die Patenterteilung: **01.12.2010**
(21) Anmeldenummer: 06015945.6
(22) Anmeldetag: 01.08.2006
(51) Int. Cl.: A61M 15/00, A61M 16/00, A61M 16/20, A61M 39/26

(54) **Ventilballon für Inhalatoren**
Breathing bag for inhalers
Ballon de ventilation pour inhalateurs.

(43) Veröffentlichungstag der Anmeldung: 06.02.2008
(73) Patentinhaber: Stobi GmbH & Co. KG, 78532 Tuttlingen (DE)
(72) Erfinder: Storz, Markus, 78532 Tuttlingen (DE)
(74) Vertreter: Späth, Dieter

(56) Entgegenhaltungen:
- EP-A1- 0 143 208
- EP-A2- 0 933 093
- WO-A-81/02982
- WO-A-2004/098689
- WO-A-2005/021074
- DE-A1- 19 546 167
- FR-A1- 2 271 845
- US-A- 3 859 997

## Beschreibung

Bei der hier vorliegenden Erfindung handelt es sich im Wesentlichen um eine Weiterentwicklung des vom selben Autor gehaltenen Patents (DE 198 03 376 bzw. EP 0933093 und US 6,513,524). Der dort beschriebene, abnehmbare Ventilballon erlaubt eine vom aerosolerzeugenden Gerät völlig unabhängige Inhalation des aroma- und wirkstoffhaltigen Aerosols. So wird eine absolut sichere Inhalation ermöglicht, da der Anwender nicht mehr mit dem in der Regel elektrisch betriebenen Aerosolerzeuger verbunden ist. Außerdem ist die Inhalation aus dem Ventilballon wesentlich komfortabler, da diese praktisch an jedem Ort und ohne die technischen Belange des Aerosolerzeugers berücksichtigen zu müssen, erfolgen kann.
Der Ventilballon eignet sich in erster Linie, jedoch nicht ausschließlich, für die Verwendung in Heißluftextraktionsinhalatoren, so genannten Verdampfern, wie sie z. B. auch im vom selben Autor gehaltenen Patent "Heißlufterzeugung von Heißluftextraktionsinhalatoren" (DE 100 42 396) beschrieben werden. Der Grund dafür liegt in der sehr geringen Tröpfchengröße des bei der thermischen Verdampfung erzeugten Aerosols im Vergleich zu Verneblern oder Versprühern, wodurch die Kondensation des Aerosols an der Ballonhülle ausreichend lange hinausgezögert wird.

### Allgemeines zur Heißluftextraktion:

Bei der Heißluftextraktion werden Heilkräuter oder andere geeignete pflanzliche Substanzen zerkleinert und mit auf bis zu 235°C erhitzter Luft durchströmt, wodurch die in den Substanzen enthaltenen Aromen und Wirkstoffe "verdampfen" bzw. ausdünsten und in die Heißluft übergehen. Diese mit Aromen und Wirkstoffen versetzte Luft wird, nachdem Sie auf eine angenehme Temperatur heruntergekühlt ist, inhaliert, wobei die Wirkstoffe über die Lungenbläschen in den Blutkreislauf gelangen. Bei Temperaturen über 235°C würden die Kräuter anfangen zu brennen, da der Selbstentzündungspunkt von Zellulose bzw. Pflanzenmaterial überschritten wäre.

Mit derartigen Inhalatoren lassen sich auch Wirkstoffe aus geeigneten Arzneimitteln in flüssiger und pulverisierter Form verdampfen und inhalieren, wobei hier durchaus Verdampfungstemperaturen weit über 235°C zur Anwendung kommen können.
Bei der Heißluftextraktion wird kein Wasser erhitzt um Wasserdampf zu erzeugen, die Dämpfe bzw. das Aerosol entstehen allein durch die Hitzeeinwirkung auf die jeweiligen Substanzen.

Der im Patent DE 198 03 376 beschriebene und seither in leicht abgewandelter Form (Verbindung von Ventil mit Füllkammer bzw. Mundstück mit wiederlösbaren Schnapphaken anstatt Schraubgewinde) verkaufte Ventilmechanismus ist teuer, schwer, empfindlich sowie aufwendig zu zerlegen und zu reinigen. Der außen am Ventil mit Gummiringen befestigte Ballon hält nicht zuverlässig. Einige Anwender sind krankheitsbedingt nicht in der Lage, den Zusammenbau der Ventilteile und das Aufziehen eines neuen Ballons zu bewerkstelligen. Für den medizinischen Einsatz wird aus hygienischen Gründen ein möglichst billiger, nach Gebrauch komplett wegwerfbarer Ventilballon benötigt.
Das Dokument DE 198 03 376 bzw. EP 09 33 093 ist der nächstliegende Stand der Technik und offenbart einen Ventilballon gemäß dem Oberbegriff des Anspruchs 1.

Der Erfindung liegt die Aufgabe zugrunde, einen möglichst preisgünstigen Ventilballon inklusive Mundstück vorzuschlagen, der eine mindestens gleich gute Funktionalität aufweist wie der bisherige.

Diese Aufgabe wird erfindungsgemäß durch die im Patentanspruch 1 aufgeführten Merkmale gelöst. Weitere vorteilhaften Ausgestaltungen der Erfindung sind in der Unteransprüchen gekennzeichnet.

Die mit der Erfindung erzielten Vorteile bestehen insbesondere darin, dass die Produktionskosten im Vergleich zu dem bisherigen Ventilmechanismus drastisch reduziert werden können. Das wiederum erlaubt die aus hygienisch-medizinischen Gründen geforderte Einmalverwendung. Außerdem ist der neue Ventilballon leichter, robuster und eine aufwendige Reinigung sowie das Aufziehen eines neuen Ballons entfallen.

Die Erfindung wird nachfolgend anhand eines in der Zeichnung dargestellten Ausführungsbeispiels näher erläutert. Es zeigen:
- Figur 1: einen Längsschnitt durch den Ventilmechanismus beim Befüllen des Ventilballons mittels Füllrohr;
- Figur 2: eine mit einem Kreuzschlitz versehene Membran in Ruhestellung und perspektivischer Ansicht;
- Figur 3: eine mit einem Kreuzschlitz versehene Membran in von einem Rohr geöffnetem Zustand und perspektivischer Ansicht;
- Figur 4: einen Längsschnitt durch den Ventilmechanismus beim Entleeren des Ventilballons mittels Mundstück;
- Figur 5: einen Längsschnitt durch den Ventilmechanismus in geschlossenem Zustand mit Mundstück;

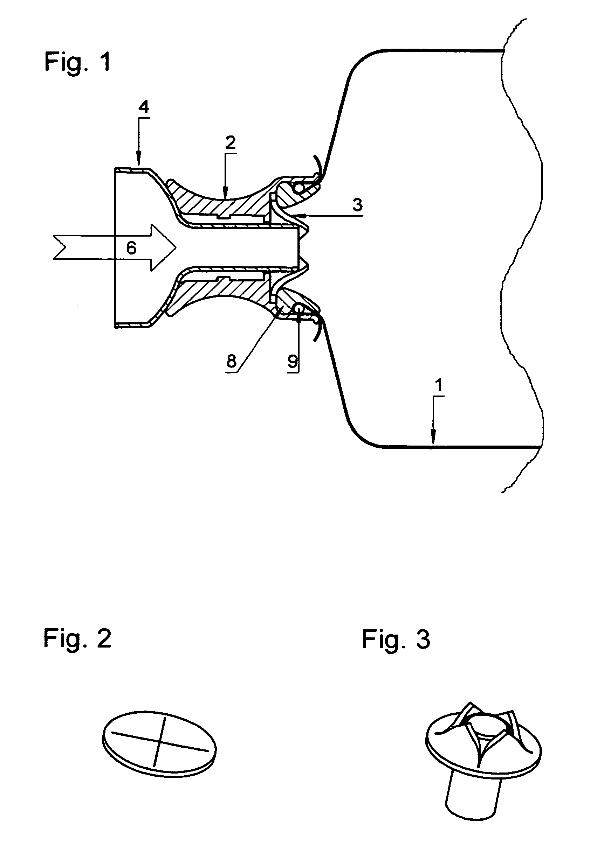

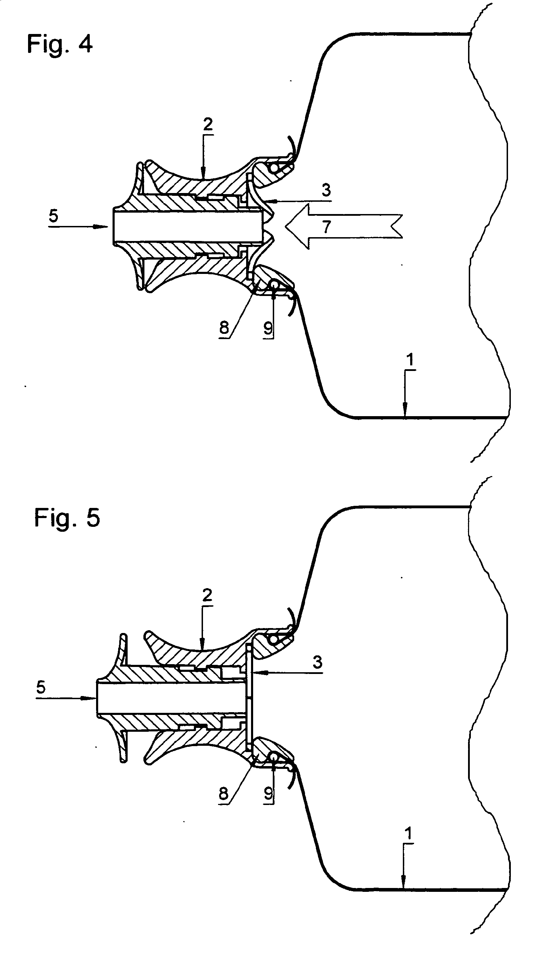

Der in Figur 1 dargestellte Ventilmechanismus weist ein Ventilgehäuse (2), den Ballon (1) in Teilansicht und die geschlitzte Membrane (3) auf. Weiter wird das Füllrohr (4) gezeigt, durch welches das Aerosol in den Ventilballon hineingepumpt wird. Die Membran (3) ist in einem vom Füllrohr (4) geöffneten Zustand. Wird das Füllrohr (4) nach dem Befüllen abgezogen, kehrt die Membrane (3) wieder in ihren ursprünglichen, flachen Zustand zurück. Sie ist dann dicht geschlossen und hält das Aerosol im Ballon.
In Figur 2 und 3 wird beispielhaft eine Membran mit Kreuzschlitz gezeigt. Es funktioniert aber auch mit einem einfachen Schlitz oder Schlitzen in beliebiger Anzahl, welche sich in der Mitte der Membran treffen.
Wie in Figur 4 dargestellt, ist jetzt anstelle des Füllrohrs das Mundstück (5) im Ventilgehäuse (2) angebracht worden. Durch Druck mit den Lippen auf das Mundstück (5) wird dieses gegen die Membran (3) geschoben, und öffnet so das Ventil wie dargestellt. Nimmt man den Ventilballon mit Mundstück von den Lippen, so schiebt die Membran (3) das Mundstück (5) selbsttätig zurück und das Ventil ist wieder geschlossen wie in Figur 5 dargestellt.
Die in Figur 1, 4 und 5 dargestellte Anbringung des Ballons (1) am Ventilgehäuse (2) weist einen elastischen O-Ring (9) auf, welcher die Ballonhülle (1) auf einem harten Klemmring (8) befestigt. Das überstehende Ende der Ballonhülle (1) wird vorzugsweise um den O-Ring (9) zurückgeschlagen, und dann zusammen mit dem Klemmring (8) in das Ventilgehäuse (2) eingerastet. Der weiche O-Ring (9) sorgt für die Abdichtung des Ballons (1) zum Ventilgehäuse (2) und zum Klemmring (8).

### Bezugszeichenliste:

| | | | |
|---|---|---|---|
| 1 | Ballon | 6 | Stromrichtung beim Befüllen |
| 2 | Ventilgehäuse | 7 | Stromrichtung beim Entleeren |
| 3 | Membran | 8 | Klemmring |
| 4 | Füllrohr | 9 | O-Ring |
| 5 | Mundstück | | |

## Patentansprüche

1. Ventilballon (1) für Inhalatoren, mit einem Ventilmechanismus zum Befüllen und Entleeren des Ventilballons (1) und mit einem rohrförmigen Mundstück (5), das zum Öffnen und Schließen eines Ventils des Ventilmechanismus in einem Ventilgehäuse (2) des Ventilmechanismus verschiebbar ist, **dadurch gekennzeichnet, dass** der Ventilmechanismus eine elastische, geschlitzte Membran (3) als Ventil aufweist, die mit dem Mundstück (5) so zusammenwirkt, dass, wenn das Mundstück (5) von einer Ausgangposition im Ventilgehäuse (2) gegen die Membran (3) geschoben wird, das Mündstück (5) das Ventil öffnet, und wenn das Mundstück (5) nicht mehr gegen die Membran (3) geschoben wird, die Membran (3) das Mundstück (5) selbsttätig in seine Ausgangposition zurückschiebt und das Ventil wieder geschlossen wird.

2. Ventilballon für Inhalatoren nach Patentanspruch 1, **dadurch gekennzeichnet, dass** die Membran (3) mittels eines einrastbaren Klemmrings (8) im Ventilgehäuse (2) befestigt wird.

3. Ventilballon für Inhalatoren nach Patentanspruch 1 oder 2, **dadurch gekennzeichnet, dass** der Ballon (1) mittels eines elastischen Rings (9) auf einem harten Klemmring (8) befestigt wird und diese Baugruppe dann innerhalb des Ventilgehäuses (2) einrastet.

## Claims

1. Valve balloon (1) for inhalers, said valve balloon having a valve mechanism for filling and emptying the valve balloon (1) and a tubular mouth piece (5), which is displaceable in a valve housing (2) of the valve mechanism for opening and closing a valve of the valve mechanism, **characterized in that** as a valve, the valve mechanism has an elastic, slotted diaphragm (3) which interacts with the mouth piece (5) such that when the mouth piece (5) is pushed from a start position in the valve housing (2) against the diaphragm (3), the mouth piece (5) opens the valve, and when the mouth piece (5) is no longer pushed against the diaphragm (3), the diaphragm (3) automatically pushes the mouth piece (5) back into the start position and the valve is closed again.

2. Valve balloon for inhalers according to Claim 1, **characterized in that** the diaphragm (3) is secured in the valve housing (2) by means of a lockable clamping ring (8).

3. Valve balloon for inhalers according to Claims 1 or 2, **characterized in that** the balloon (1) is secured on a hard clamping ring (8) by means of an elastic ring (9) and this assembly then locks into position inside the valve housing (2).

## Revendications

1. Ballon à valve (1) pour inhalateurs, avec un mécanisme à valve pour remplir et vider le ballon à valve (1), et avec un embout tubulaire (5) qui peut coulisser dans un logement de valve (2) du mécanisme à valve afin d'ouvrir et de fermer une valve du mécanisme à valve, **caractérisé en ce que** le mécanisme à valve présente comme valve une membrane élastique fendue (3), qui coopère avec l'embout (5) de telle sorte que, lorsque l'embout (5) est poussé contre la membrane (3) dans le logement de valve (2) à partir d'une position de départ, l'embout (5) ouvre la valve, et que, lorsque l'embout (5) n'est plus poussé contre la membrane (3), la membrane (3) repousse automatiquement l'embout (5) dans sa position de départ et la valve est refermée.

2. Ballon à valve pour inhalateurs selon la revendication 1, **caractérisé en ce que** la membrane (3) est fixée dans le logement de valve (2) au moyen d'une bague de serrage enclenchable (8).

3. Ballon à valve pour inhalateurs selon la revendication 1 ou 2, **caractérisé en ce que** le ballon (1) est fixé au moyen d'une bague élastique (9) sur une bague de serrage dure (8), et cet ensemble s'enclenche ensuite à l'intérieur du logement de valve (2).
